# EUROPÄISCHE PATENTSCHRIFT

(11) **EP 0 789 694 B1**
(45) Veröffentlichungstag und Bekanntmachung des Hinweises auf die Patenterteilung: **09.04.2003**
(21) Anmeldenummer: 95936561.0
(22) Anmeldetag: 27.10.1995
(51) Int. Cl.: C07D 307/92, C07B 41/04

(54) **VERFAHREN ZUR HERSTELLUNG VON 8-ALPHA,12-OXIDO-13,14,15,16-TETRANORLABDAN**
METHOD FOR PREPARING 8-ALPHA,12-OXIDO-13,14,15,16-TETRANORLABDANUM
PROCEDE DE PRODUCTION DE 8-ALPHA,12-OXYDO-13,14,15,16-TETRANORLABDANUM

(30) Priorität: 05.11.1994 DE 4439574
(43) Veröffentlichungstag der Anmeldung: 20.08.1997
(73) Patentinhaber: Cognis Deutschland GmbH & Co. KG, 40589 Düsseldorf (DE)
(72) Erfinder: KNÜBEL, Georg, D-40229 Düsseldorf (DE); BOMHARD, Andreas, D-40591 Düsseldorf (DE); MARKERT, Thomas, D-40591 Düsseldorf (DE)
(86) Internationale Anmeldenummer: EP9504225
(87) Internationale Veröffentlichungsnummer: WO96014310

(56) Entgegenhaltungen:
- EP-A- 0 170 955
- EP-A- 0 212 254
- EP-A- 0 550 889
- WO-A-90/12793
- GB-A- 701 911
- US-A- 3 029 255
- TETRAHEDRON, Bd. 49, Nr. 28, 1993 OXFORD, Seiten 6251-6262, BARRERO,A.F. ET AL. 'Synthesis of Ambrox from Communic Acids' in der Anmeldung erwähnt
- J.ORG.CHEM., Bd. 57, Nr. 3, 31.Januar 1992 Seiten 955-960, SNOWDEN,R.L. ET AL. 'Internal Nucleophilic Termination in Biomimetic Acid Mediated Polyene Cyclisations: Sterochemical and Mechanistic Implications. Synthesis of(+-)-Ambrox and Its Diastereomers '
- CHEMISTRY AND INDUSTRY, 20.August 1990 LONDON, Seiten 516-520, SELL,C. 'The Chemistry of Ambergris'

## Beschreibung

### Gebiet der Erfindung

Die Erfindung betrifft ein Verfahren zur Herstellung von 8α,12-Oxido-13,14,15,16-tetranorlabdan.

### Stand der Technik

8α,12-0xido-13,14,15,16-tetranorlabdan, fortan als **Ambroxan** bezeichnet, ist ein wertvoller Ambrariechstoff, der in der Ambra, einer Stoffwechselausscheidung des Pottwals, enthalten ist (Ullmanns Encyklopädie der technischen Chemie, Band 20, Seite 283, Verlag Chemie Weinheim 1981). Synthetisch kann Ambroxan aus Sclareol durch oxidativen Seitenkettenabbau und nachfolgende Reduktion des gebildeten Lactons (**Sclareolid**) gemäß US 30 50 532 hergestellt werden. Die Überführung von Sclareolid in das geruchlose 8α,12-Dihydroxy-13,14,15,16-tetranorlabdan, fortan kurz als **Diol** bezeichnet, erfolgt in an sich bekannter Weise, beispielsweise durch Reduktion mit Lithiumaluminiumhydrid (Helv. Chim. Acta 1950, 33, 1310), mit Natriumborhydrid (Chem. Abstr. 57, 7316a) oder mit Kaliumborhydrid/Lithiumchlorid-Mischungen (Chem. Abstr. 94, 15913q).

Die cyclisierende Dehydratisierung des Diols zu Ambroxan kann mit sauren Katalysatoren, beispielsweise p-Toluolsulfonsäure, p-Toluolsulfonsäurechlorid, katalytischen Mengen an Schwefelsäure sowie sauren Ionenaustauschern in verschiedenen Lösungsmitteln, beispielsweise Toluol, Hexan, Pyridin, Tetrahydrofuran oder Methanol, vorzugsweise bei Siedetemperatur, durchgeführt werden.

In US 3 029 255 wird die Verwendung von β-Naphthalinsulfonsäure oder Tonerde als Dehydratisierungskatalysatoren bei der Herstellung von Ambroxan beschrieben. Bei diesem Verfahren werden neben Verharzungsprodukten und Olefinen weitere Nebenprodukte erhalten, so daß die Ausbeute an Ambroxan weniger als 77 % beträgt.

JP-A-86/33184 (Takasago) beschreibt ein Verfahren zur Herstellung von Ambroxan, bei der die Cyclisierung der Diolvorstufe durch spezielle Katalysatoren induziert wird. Bei diesen Katalysatoren handelt es sich um säure-beladene aktive Weißerde, Tonerde oder Kieselerde. Als Säuren werden dabei insbesondere Schwefelsäure, Phosphorsäure und Polyphosphorsäure offenbart. Das Takasago-Verfahren hat jedoch Nachteile bezüglich
a) des Umsatzes an Edukt, d. h. an eingesetztem Diol und/oder
b) der Bildung an Dehydratisierungsprodukten (Nebenprodukten) und/oder
c) der Stereoselektivität der Ringschlußreaktion (Ausmaß der iso-Ambrox-Bildung).

Die genannten Nachteile des Takasago-Verfahrens werden durch das in der jüngeren Anmeldung WO 90/12793 beschriebene Verfahren der Anmelderin vermieden. Das Verfahren gemäß WO 90/12793 erfordert jedoch relativ hohe Reaktionstemperaturen. Darüber hinaus läßt sich die Bildung von Dehydratisierungsprodukten nicht vollständig vermeiden.

Insgesamt ist festzustellen, daß bei den aus dem Stande der Technik bekannten Verfahren zur Herstellung von Ambroxan durch Cyclisierung der Diolvorstufe in mehr oder weniger hohem Maße Dehydratisierungsprodukte auftreten. Bei diesen Dehydratisierungsprodukten handelt es sich nach Untersuchungen der Anmelderin um Verbindungen, die durch die Formeln (I), (II) und (III) beschrieben werden können.

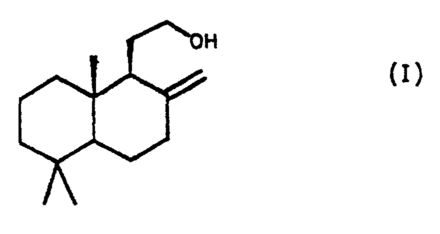

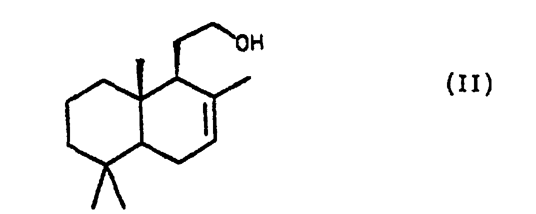

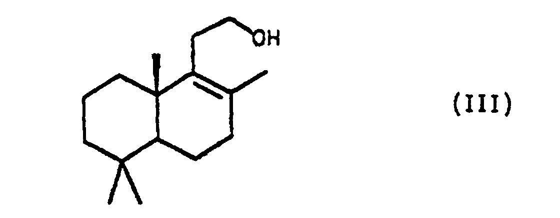

Die genannten Dehydratisierungsprodukte sind geruchlos und mindern die Ausbeute an Ambroxan, das bei der sauren Cyclisierung der Diolvorstufe erhalten wird.

Aus der EP-A-212 254 ist bekannt, daß die Verbindung der Formel (I) sauer zu Ambroxan cyclisiert werden kann. Die Beschreibung der Patentanmeldung offenbart jedoch keinerlei Hinweise darüber, in welcher Weise eine solche Cyclisierung durchzuführen ist. Insbesondere enthält sie keine Hinweise darauf, welche kritischen Parameter eingehalten werden müssen, um sicherzustellen, daß die Cyclisierung von (I) in effizienter Weise erfolgt.

A.F. Barrero et al. haben die Cyclisierung zweier Derivate von (I) unter saurer Katalyse untersucht (vergleiche: Tetrahedron, 1993, Vol. 49, No. 28, S. 6251-6262). Diese Derivate unterscheiden sich strukturell dadurch von der Verbindung (I), daß eine der in (I) vorhandenen geminalen Methylgruppen durch eine Gruppe -COOMe oder -CH₂OH ersetzt ist. Die Cyclisierung funktioniert gut bei 0 °C in dem sehr polaren Lösungsmittel Nitromethan. Bei höheren Temperaturen wird jedoch bevorzugt 8-epi-Ambroxan gebildet.

Die Cyclisierung der Verbindungen (II) und (III) ist unseres Wissens in der Literatur nicht beschrieben. Sie ist für den Fachmann aus der Kenntnis der EP-A-212 254 und der genannten Publikation von A.F. Barrero et al. auch nicht nahegelegt. Dem Fachmann ist nämlich bekannt, daß Reaktionen, die auf eine bestimmte Verbindung mit gutem Erfolg anwendbar sind, nicht zwangsläufig mit demselben Erfolg auf isomere oder analoge Verbindungen übertragen werden können. Dies ist insbesondere auch für die Herstellung von Stereoisomeren des Ambroxans bekannt. So haben beispielsweise P.F.Vlad und N.D.Ungur die Herstellung des 8-epi- und des 9-epi-Ambroxans und die des Ambroxans durch Ringschluß der entsprechenden 1,4-Diol-Vorstufen mit Dimethylsulfoxid/Chlortrimethylsilan untersucht (vergleiche: Synthesis 1983, Seiten 216-219). Sie fanden, daß die Ausbeute bei der Cyclisierung in hohem Maße von der Stereochemie des Eduktes abhängig ist: Während Ambroxan und sein 9-epi-Isomer mit 85%- bzw. 90%-iger Ausbeute erhalten wurden, fiel das 8-epi-Isomer nur mit 46%-iger Ausbeute an.

### Beschreibung der Erfindung

Die Aufgabe der vorliegenden Erfindung bestand darin, ein Verfahren zur Cyclisierung eines Gemisches der Verbindungen (I), (II) und (III) zu entwickeln, das in effizienter Weise Ambroxan durch saure Cyclisierung zugänglich macht. Eine weitere Aufgabenstellung der vorliegenden Erfindung bestand darin, daß das beim Ringschluß gebildete Ambroxan die geruchlich wertlosen Epimeren (8-epi- bzw. 9-epi-Ambroxan) nur in untergeordneten Mengen enthält. Unter dem Begriff "untergeordnete Menge" wird im Rahmen der vorliegenden Erfindung verstanden, daß die Epimeren des Ambroxans - bezogen auf die Gesamtmenge an Ambroxan, 8-epi- und 9-epi-Ambroxan - in der Reaktionsmischung nach der Cyclisierung zu maximal 30 Gew.-% vorliegen.

Diese Aufgabe wurde erfindungsgemaß gelöst durch ein Verfahren zur Herstellung von 8α,12-Oxido-13,14,15,16-tetranorlabdan durch Cyclisierung eines Gemisches der Verbindungen I, II und III, worin die Verbindungen I bis III die oben näher bezeichneten Strukturformeln haben. Die Cyclisierung wird dabei erfindungsgemäß in Gegenwart von 10 bis 100 Gew.-% - bezogen auf das eingesetzte Gemisch der Verbindungen I bis III - von Methansulfonsäure durchgeführt.

Gegenstand der Erfindung ist dementsprechend ein Verfahren zur Herstellung von 8α,12-Oxido-13,14,15,16-tetranorlabdan durch Cyclisierung eines Gemisches der Verbindungen I bis III in Gegenwart von 10 bis 100 Gew.-% - bezogen auf das eingesetzte Gemisch der Verbindungen I bis III - von Methansulfonsäure. Dabei soll das Gemisch der Verbindungen I bis III jede der Komponenten I bis III zu mindestens 10 Gew.-% - bezogen auf das gesamte Gemisch der Verbindungen I bis III - enthalten.

Die Reaktionstemperatur für die saure Cyclisierung ist bei dem erfindungsgemäßen Verfahren an sich nicht kritisch. Es ist jedoch bevorzugt, die Reaktion im Temperaturbereich von -10 bis 30 °C durchzufühen; dadurch ist der Tatsache Rechnung getragen, daß einerseits eine für praktische Zwecke ausreichende Reaktionsgeschwindigkeit gewährleistet ist, andererseits eine unerwünschte Epimerisierung der Zielsubstanz unterbleibt. Besonders bevorzugt ist es, die Reaktion im Temperaturbereich von 0 bis 20 °C durchzuführen.

Der Cyclisierungskatalysator wird in dem erfindungsgemäßen Verfahren in einer Menge von - bezogen auf das Diol - 10 bis 100 Gew.-% eingesetzt. Die bevorzugte Menge liegt im Bereich von 30 bis 60 Gew.-%.

In dem erfindungsgemäßen Verfahren setzt man als Cyclisierungskatalysator Methansulfonsäure ein.

Geeignete Lösungsmittel für das erfindungsgemäße Verfahren sind beispielsweise Toluol und/oder Xylol sowie halogenierte Kohlenwasserstoffe. Besonders bevorzugt als Lösungsmittel ist Dichlormethan.

Die folgenden Beispiele dienen der Erläuterung der Erfindung und sind nicht einschränkend zu verstehen.

### Beispiele

### 1. Allgemeines

### 1.1. Herstellung eines Gemisches der Verbindungen I bis III

100 g 8α,12-Dihydroxy-13,14,15,16-tetranorlabdan wurden in 500 ml Acetanhydrid unter Rühren vorgelegt. Nach Zugabe von 30 Tropfen konzentrierter Schwefelsäure setzte unter Verfärbung die Acetylierung ein, wobei die Temperatur auf ca. 50 °C anstieg. Nach 7 Stunden wurde mit 300 ml H₂O versetzt und dreimal mit je 100 ml Methyl-t-butylether extrahiert. Die vereinigten Etherextrakte wurden mit Natriumhydrogencarbonat-Lösung gewaschen und über Natriumsulfat getrocknet. Abschließend destillierte man bei 150 °C und 0,04 mbar. Die so gewonnene Hauptfraktion (75 g) wurde dann unter Rühren mit 25 g Kaliumhydroxid, gelöst in 200 ml Methanol, versetzt. Nach 24 Stunden unter Rühren gab man 300 ml Wasser zu und extrahierte dreimal mit jeweils 200 ml Dichlormethan. Im Anschluß wurde die organische Phase in Vakuum eingeengt. Die Ausbeute (Summe der Verbindungen I bis III) betrug 64 g. Laut ¹³C-NMR lagen die Isomeren I, II und III im Verhältnis 2:3,5:3 vor.

### 1.2. Analytik

Zur Quantifizierung der Produkte wurde die gaschromatographische Analyse herangezogen (50 m WG11- Quartz-Kapillare; Injektortemperatur: 220 °C; Detektortemperatur: 250 °C; Ofentemperatur: 80 -> 220 °C bei einer Aufheizgeschwindigkeit von 8 °C/min; Trägergas: Stickstoff; Druck: 20 psi).

### 2. Versuchsbeschreibungen

### Beispiel 1 (zum Vergleich)

2,5 g des Gemisches der Verbindungen I bis III (vergl. Nr. 1.1) wurden in 25 ml Dichlormethan gelöst und mit 1,6 g p-Toluolsulfonsäure versetzt. Nach 7-stündigem Rühren bei Raumtemperatur zeigte das Gaschromatogramm der Reaktionsmischung 63 % Ambroxan und 20 % 8-epi-Ambroxan.

### Beispiel 2 (erfindungsgemäß)

Beispiel 1 wurde wiederholt, wobei jedoch als Katalysator 0,8 g Methansulfonsäure eingesetzt wurden. Nach 2-stündigem Rühren bei Raumtemperatur enthielt die Reaktionsmischung 83 % Ambroxan und 6 % 8-epi-Ambroxan.

## Patentansprüche

1. Verfahren zur Herstellung von 8α,12-Oxido-13,14,15,16-tetranorlabdan durch Cyclisierung eines Gemisches der Verbindungen (I), (II) und (III) 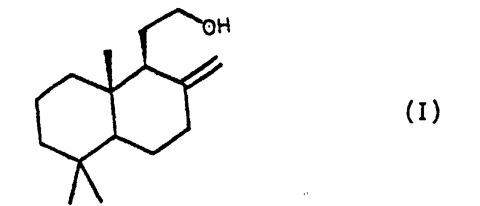 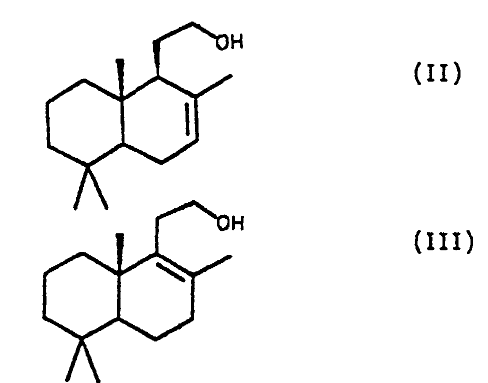 **dadurch gekennzeichnet, daß** man die Cyclisierung in Gegenwart von 10 bis 100 Gew.-% - bezogen auf das eingesetzte Gemisch (I) bis (III) - von Methansulfonsäure durchführt.

2. Verfahren nach Anspruch 1, wobei man die Säure in einer Menge von - bezogen auf das eingesetzte Gemisch der Verbindungen (I) bis (III) - 30 bis 60 Gew.-% einsetzt.

3. Verfahren nach Anspruch 1 oder 2, wobei man die Cyclisierung bei Temperaturen im Bereich von -10 bis 30 °C durchführt.

4. Verfahren nach einem der Ansprüche 1 bis 3, wobei man die Cyclisierung in Dichlormethan durchführt.

## Claims

1. A process for the production of 8α,12-oxido-13,14,15,16-tetranorlabdane by cyclization of a mixture of compounds (I), (II) and (III): 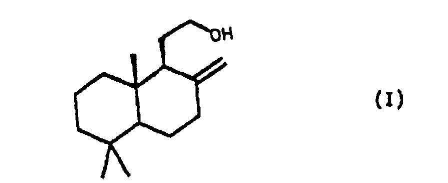 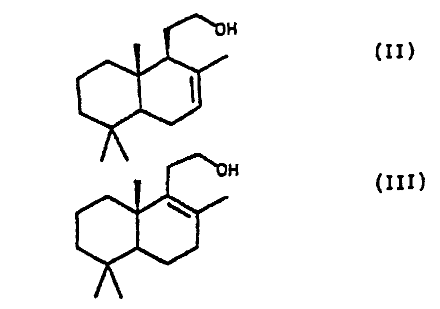 **characterized in that** the cyclization is carried out in the presence of 10 to 100% by weight - based on the mixture (I) to (III) used - of methanesulfonic acid.

2. A process as claimed in claim 1, **characterized in that** the acid is used in a quantity of - based on the mixture of compounds (I) to (III) used - 30 to 60% by weight.

3. A process as claimed in claim 1 or 2, **characterized in that** the cyclization is carried out at temperatures of -10 to 30°C.

4. A process as claimed in any of claims 1 to 3, **characterized in that** the cyclization is carried out in dichloromethane.

## Revendications

1. Procédé de préparation de 8α, 12-oxydo-13, 14, 15, 16-tétranorlabdane,
par cyclisation d'un mélange de composés (I), (II) et (III) : 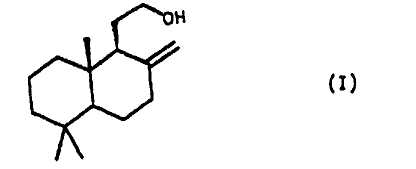 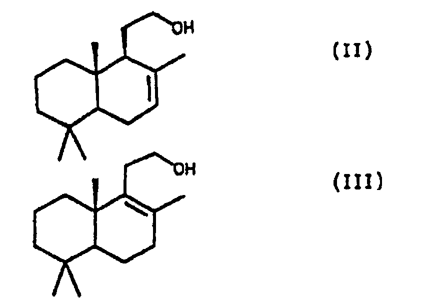 **caractérisé en ce qu'**
on effectue la cyclisation en présence de 10 à 100 % en poids, d'acide méthane sulfonique rapporté au mélange utilisé (I) à (III).

2. Procédé selon la revendication 1,
dans lequel
on met en oeuvre l'acide en une quantité de 30 à 60 % en poids rapporté au mélange mis en oeuvre des composés (I) à (III).

3. Procédé selon la revendication 1 ou la revendication 2,
dans lequel
on effectue la cyclisation à des températures dans la zone de -10° à 30°C.

4. Procédé selon l'une des revendications 1 à 3,
dans lequel on effectue la cyclisation dans le dichlorométhane.
